# EUROPÄISCHE PATENTSCHRIFT

(11) **EP 4 117 769 B1**
(45) Veröffentlichungstag und Bekanntmachung des Hinweises auf die Patenterteilung: **18.11.2026**
(21) Anmeldenummer: 21711809.0
(22) Anmeldetag: 09.03.2021
(51) Int. Cl.: A61M 39/10, A61M 39/20, F16L 29/00, F16L 37/12, F16L 47/00, F16L 37/38

(54) **KUPPLUNGSELEMENT FÜR EIN GESCHLOSSENES FLUIDTRANSFERSYSTEM, AUFSATZ FÜR EIN SOLCHES KUPPLUNGSELEMENT, KUPPLUNGSSYSTEM SOWIE KUPPLUNGSKIT**
COUPLING ELEMENT FOR A CLOSED FLUID TRANSFER SYSTEM, ATTACHMENT FOR A COUPLING ELEMENT OF THIS TYPE, COUPLING SYSTEM, AND COUPLING KIT
ÉLÉMENT DE RACCORD POUR UN SYSTÈME DE TRANSFERT DE FLUIDE FERMÉ, ACCESSOIRE POUR UN ÉLÉMENT DE RACCORD DE CE TYPE, SYSTÈME DE RACCORD, ET KIT DE RACCORD

(30) Priorität: 11.03.2020 DE 102020203153
(43) Veröffentlichungstag der Anmeldung: 18.01.2023
(73) Patentinhaber: B. Braun Melsungen AG, 34212 Melsungen (DE)
(72) Erfinder: BERG, Karl Martin, 34212 Melsungen (DE); FISCHER, Nathanael, 36251 Bad Hersfeld (DE); SEIDEL, Gerrit, 34121 Kassel (DE); KOPP, Florin, 26419 Schortens (DE)
(74) Vertreter: Maiwald GmbH
(86) Internationale Anmeldenummer: PCT/EP2021/055842
(87) Internationale Veröffentlichungsnummer: WO 2021/180675

(56) Entgegenhaltungen:
- EP-A1- 2 589 367
- WO-A1-2018/136361
- US-A1- 2016 136 412

## Beschreibung

Die Erfindung betrifft ein Kupplungselement für ein geschlossenes Fluidtransfersystem, ein Kupplungssystem sowie ein Kupplungskit.

Viele Substanzen, die als Injektionen oder in vergleichbarer Zuführungsform verabreicht werden, wie CMR-Arzneimittel, die beispielsweise in der Krebstherapie eingesetzt werden und bei ihrer therapeutischen Anwendung vor allem auf die Schädigung wachstumsintensiver Tumorzellen gerichtet sind, weisen außerhalb der eigentlichen Therapieanwendung ein erhebliches Gefährdungspotential auf. So sind solche Substanzen bedingt durch ihren Wirkmechanismus zum Teil selbst krebserzeugend, weshalb der Kontakt mit nicht in Therapie befindlichen Personen zu vermeiden ist. Für CMR-Arzneimittel werden daher bei der Herstellung applikationsfertiger Zubereitungen vermehrt geschlossene Medikamententransfersystem, sogenannte "Closed System Transfer Devices" oder auch CSTDs eingesetzt. Wichtiger Bestandteil solcher CSTDs sind Kupplungssysteme, die den sicheren Transfer von CMR-Arzneimitteln oder anderen Substanzen ermöglichen und nach dem Trennen der Verbindung trocken abschließen, um so die Umgebung vor einer Kontamination, z.B. durch Leckagen oder Tropfenbildung auf den Oberflächen der Kupplungselemente, zu schützen.

Kupplungssysteme dieser Art werden im Allgemeinen mit den Begriffen "dry connection", "automatic self-sealing technology" oder "closed connection" in Verbindung gebracht und essentiell für die Realisierung geschlossener Fluidtransfersysteme.

Die WO 2018/136361 A1 betrifft einen Spritzenadapter, der ein Gehäuse, eine Kanüle mit einem ersten Ende und einem zweiten Ende, wobei das zweite Ende der Kanüle innerhalb des Gehäuses positioniert ist, und eine Klemmhülse mit einem ersten Ende und einem zweiten Ende, wobei mindestens ein Abschnitt der Klemmhülse innerhalb des Gehäuses aufgenommen ist, umfasst. Die Hülse umfasst einen Körper, der einen Durchgang mit einer Membran definiert, die von dem Durchgang aufgenommen wird.

Die EP 2 589 367 A1 betrifft einen Steckverbinder mit einem Verbinderhauptkörper, der aus einem Außenrohr mit einer zylindrischen Form, einem Innenrohr mit einer zylindrischen Form und einer Nabe, einer Hohlnadel, die an der Nabe gelagert ist, einem ersten Dichtungselement, das an dem Innenrohr gelagert ist, einer Schraubenfeder, die als Einstichabschnittsvorspannmittel zum Vorspannen des ersten Dichtungselements in einer Richtung zu dessen distalem Ende dient, und einem Klemmelement, das an dem Außenrohr angeordnet ist, konfiguriert ist.

Die US 2016/136412 A1 betrifft eine Verbindungsvorrichtung für eine medizinische Vorrichtung, die ein erstes Verbindungselement, das ein erstes Ende und ein zweites Ende aufweist, wobei das erste Verbindungselement mindestens einen Kanal neben dem ersten Ende definiert, umfasst. Der mindestens eine Kanal hat einen ersten Abschnitt, der sich in einer axialen Richtung erstreckt, und eine Schulter benachbart zu dem ersten Ende des ersten Verbindungselements, die einen Kanaleintritt definiert. Der mindestens eine Kanal weist einen zweiten Abschnitt auf, der sich in einer Querrichtung relativ zu der axialen Richtung erstreckt. Die Verbindungsvorrichtung umfasst ferner ein zweites Verbindungselement mit einem ersten Ende und einem zweiten Ende und mindestens einem Vorsprung benachbart zu dem ersten Ende. Die Schulter ist dazu ausgebildet, den mindestens einen Vorsprung des zweiten Verbindungselements unabhängig von der Ausrichtung des mindestens einen Vorsprungs relativ zu dem Kanaleingang in den Kanaleingang zu führen.

Bekannte Kupplungssysteme weisen Kupplungselemente mit jeweils einer weiblichen und einer männlichen Kupplungsseite oder anderweitig unterschiedlichen Kupplungsseiten auf, so dass für solche Kupplungssysteme zur Konnektierung unterschiedliche Kupplungselemente vorgehalten werden müssen. Dies hat nicht nur Einfluss auf die Vorhaltung, sondern gleichsam auf die Herstellung entsprechender Kupplungssysteme. Zudem sind für solche Kupplungselemente Adapter vorzusehen, um beispielsweise das Aneinanderkoppeln von Spritzen zu ermöglichen.

Ein weiterer Nachteil der bekannten Kupplungssysteme bzw. der damit verbundenen Kupplungselemente ist die schlechte Zugänglichkeit der Kupplungsoberflächen, die bei einer Konnektierung miteinander verbunden werden, wodurch eine Desinfektion dieser Flächen erschwert wird.

In Anbetracht der mit dem Stand der Technik verbundenen Nachteile ist es Aufgabe der vorliegenden Erfindung, ein Kupplungselement, einen Aufsatz für ein solches Kupplungselement, ein Kupplungssystem sowie ein Kupplungskit bereitzustellen, die eine verbesserte Handhabung aufweisen.

Die erfindungsgemäße Aufgabe wird durch ein Kupplungselement nach Anspruch 1 und durch ein Kupplungssystem nach Anspruch 10 sowie ein Kupplungskit nach Anspruch 14 gelöst. Weitere vorteilhafte Ausgestaltungen der Erfindung ergeben sich aus den Unteransprüchen.

In einem Aspekt stellt die folgenden Offenbarung ein Paar Kupplungselemente für ein gemeinsames geschlossenes Fluidtransfersystem zur Verfügung, wobei jedes Kupplungselement des Paares von Kupplungselementen umfasst: ein Kupplungsgehäuse mit einer Längsachse und mit einem Fluidanschluss und einer Kupplungsseite, eine in dem Kupplungsgehäuse angeordnete Dichtelementaufnahme mit zumindest einer fluiddichten Kammer, die durch zumindest ein in der Dichtelementaufnahme angeordnetes Dichtelement ausgebildet ist, sowie einen in dem Kupplungsgehäuse in einer Dornaufnahme exzentrisch angeordneten Dorn, zur fluiden Verbindung mit dem Fluidanschluss, wobei die Kupplungselemente jeweils miteinander komplementär, insbesondere baugleich sind und durch einen relativen Winkelversatz um ihre Längsachse, bevorzugt durch einen relativen Winkelversatz um 90°, ohne Adapter koppelbar sind.

In einem weiteren Aspekt stellt die folgende Offenbarung ein Kupplungselement für ein geschlossenes Fluidtransfersystems zur Verfügung, welches umfasst: ein Kupplungsgehäuse mit einem Fluidanschluss und einer Kupplungsseite, eine in dem Kupplungsgehäuse angeordnete Dichtelementaufnahme mit zumindest einer fluiddichten Kammer, die durch zumindest ein in der Dichtelementaufnahme angeordnetes Dichtelement ausgebildet ist, sowie einen in dem Kupplungsgehäuse in einer Dornaufnahme exzentrisch angeordneten Dorn, zur fluiden Verbindung mit dem Fluidanschluss, wobei die Dichtelementaufnahme in einer Richtung entlang des Dorns relativ zum Dorn bewegbar ist und/oder der Dorn in seiner Längsrichtung relativ zur Dichtelementaufnahme bewegbar ist, und wobei der Dorn ausgehend von einer der Dornaufnahme abgewandten Dornspitze zumindest eine vordere Fluidöffnung und eine hintere Fluidöffnung umfasst, wobei die vordere Fluidöffnung so positioniert ist, dass sie in einer der Dornaufnahme zugewandten Bewegungsendposition der Dichtelementaufnahme und/oder in einer der Kupplungsseite zugewandten Bewegungsendposition des Dorns über die der Dornaufnahme abgewandten Seite der fluiddichten Kammer hinausragt und in einer der Dornaufnahme abgewandten Bewegungsendposition der Dichtelementaufnahme und/oder in einer der Kupplungsseite abgewandten Bewegungsendposition des Dorns in der fluiddichten Kammer angeordnet ist.

Das Kupplungselement weist gemäß vorstehender Konfiguration somit einen durch die Relativbewegung zwischen Dorn und Dichtelementaufnahme bedingten dekonnektierten Zustand, in dem sich die Dichtelementaufnahme in einer der Kupplungsseite zugewandten Bewegungsendposition und/oder der Dorn in einer der Dornaufnahme zugewandten Bewegungsendposition befindet, und einen konnektierten Zustand, in dem sich die Dichtelementaufnahme in einer der Dornaufnahme zugewandten Bewegungsendposition und/oder der Dorn in einer der Kupplungsseite zugewandten Bewegungsendposition befindet, auf. Der dekonnektierte Zustand bezieht sich auf einen Ausgangszustand des Kupplungselements ohne äußere Krafteinwirkung. Unter einem konnektierten Zustand wird der Zustand verstanden, in dem ein Fluidaustausch mit einem weiteren Kupplungselement oder einem Aufsatz vorsehbar ist. Entsprechend ragt die vordere Fluidöffnung im konnektierten Zustand über die der Dornaufnahme abgewandten Seite der fluiddichten Kammer hinaus, um zum Fluidaustausch in ein weiteres Kupplungselement oder einen Aufsatz bewegt werden zu können.

Die hintere Fluidöffnung ist dazu vorgesehen, auch einen Fluidaustausch mit einem in das Kupplungselement eingreifenden Dorn eines weiteren Kupplungselements oder Aufsatzes im konnektierten Zustand ermöglichen zu können. Mit anderen Worten kann über die vordere Fluidöffnung und/oder über die hintere Fluidöffnung ein Fluidaustausch erfolgen. Somit können beispielsweise komplementäre Kupplungselemente verwendet werden, da die jeweils vordere Fluidöffnung des einen Dorns mit der jeweils hinteren Fluidöffnung des anderen Dorns zusammenwirken kann, um eine fluide Verbindung ausbilden zu können. Wäre jeweils nur eine vordere Fluidöffnung vorgesehen, so wird im konnektierten Zustand keine fluide Verbindung geschaffen. Wäre jeweils nur eine vordere Fluidöffnung des einen Dorns und eine hintere Fluidöffnung des anderen Dorns vorgesehen, so wären wieder unterschiedliche Kupplungselemente vorzuhalten.

Der Begriff "komplementär" bezieht sich auf die zwei Kupplungselemente des Paares von Kupplungselementen, insbesondere bezieht sich der Begriff "komplementär" auf eine zur Konnektierung zum Fluidaustausch funktionsgleiche Ausgestaltung. Beispielsweise können komplementäre Kupplungselemente Unterschiede in ihren nicht zur fluiden Verbindung vorgesehenen Komponenten oder Unterschiede, die keine Auswirkungen auf die fluide Verbindung als solche haben aufweisen. Hierdurch kann die Kompatibilität erhöht oder die Variantenanzahl verringert werden. Zur weiteren Reduzierung der Variantenvielzahl können auch baugleiche Kupplungselemente vorgesehen werden. Der Begriff "baugleich" bezieht sich auf die zwei Kupplungselemente des Paares von Kupplungselementen, die zumindest im notwendigen Kupplungsbereich baugleich sind.

Im Sinne einer Begünstigung der Kompatibilität ist der Dorn des Kupplungselements in dem Kupplungsgehäuse exzentrisch angeordnet, so dass der Dorn beispielsweise bei einer Konnektierung mit einem weiteren Kupplungselement an dem Dorn dieses weiteren Kupplungselements vorbeigeführt werden kann.

In einer Ausgestaltung des Kupplungselements ist die zumindest eine hintere Fluidöffnung in einer der Dornaufnahme zugewandten Bewegungsendposition der Dichtelementaufnahme und/oder in einer Kupplungsseite zugewandten Bewegungsendposition des Dorns in der fluiddichten Kammer angeordnet.

Demnach ist die hintere Fluidöffnung im konnektierten Zustand in der fluiddichten Kammer angeordnet, so dass hierüber keine Fluide auf der Kupplungsseite austreten können. Der Austritt von fluiden kann somit bei sachgerechtem Zustand des Kupplungselements nur über die vordere Fluidöffnung erfolgen. Mögliche Sicherungsmaßnahmen können folglich auf die vordere Fluidöffnung beschränkt werden.

In einer Weiterbildung ist die fluiddichte Kammer aus zwei Dichtelementen gebildet, wobei das der Dornaufnahme zugewandte Dichtelement bevorzugt mit dem Dorn in positionsfester Beziehung steht und die Dichtelementaufnahme hierzu relativ zum Dorn bewegbar ist.

Sofern die fluiddichte Kammer nicht über ein Dichtelement oder das Zusammenwirken eines Dichtelements mit dem Kupplungsgehäuse, sondern über zwei Dichtelemente ausgebildet wird, kann die Ausgestaltung der Dichtelemente vereinfacht und/oder das Volumen der fluiddichten Kammer flexibel eingestellt werden.

Wenn das der Dornaufnahme zugewandte Dichtelement mit dem Dorn in positionsfester Beziehung steht, wobei die hintere Fluidöffnung folglich auf einer der Dornaufnahme abgewandten Seite des der Dornaufnahme zugewandten Dichtelements angeordnet ist, wird das Volumen der fluiddichten Kammer ausgehend von einem dekonnektierten Zustand bei einer Konnektierung durch Bewegung der Dichtelementaufnahme mit dem der Kupplungsseite zugewandten Dichtelement verringert. Dadurch kann das Volumen der fluiddichten Kammer im konnektierten Zustand auf ein zum Fluidaustausch erforderliches Minimalvolumen reduziert werden. Somit wird gleichzeitig die Menge des nicht direkt ausgetauschten Fluids, was bei einer Dekonnektierung in der fluiddichten Kammer verbleiben könnte, verringert.

Insbesondere umfasst das Kupplungsgehäuse ein komprimierbares Dichtelement, das die Dornaufnahme mit der Dichtelementaufnahme fluiddicht verbindet.

Durch das komprimierbare Dichtelement kann die hintere Fluidöffnung in einem dekonnektierten Zustand auch außerhalb der fluiddichten Kammer angeordnet werden, da auch im Bereich der hinteren Fluidöffnung verbleibende Fluidrückstände nicht aus dem Kupplungsgehäuse austreten können. In dieser Konfiguration können beispielsweise ein oder zwei die fluiddichte Kammer ausbildende Dichtelemente positionsfest in der Dichtelementaufnahme angeordnet sein. Das eine oder die zwei Dichtelemente sind derart in der Dichtelementaufnahme positioniert, dass die hintere Fluidöffnung im konnektierten Zustand, also in einer der Dornaufnahme zugewandten Bewegungsendposition der Dichtelementaufnahme, in der durch das eine oder die zwei Dichtelemente gebildeten fluiddichten Kammer liegt.

Das komprimierbare Dichtelement kann aus einem elastischen Material gebildet sein und/oder eine teleskopartige Struktur aufweisen.

In einer weiteren Ausgestaltung weist das Kupplungselement in einer zur Erstreckungsrichtung des Dorns radialen Umfangsfläche in Umfangsrichtung zumindest zwei Befestigungsabschnitte auf, die jeweils für den Eingriff eines Befestigungselements ausgebildet sind, und wobei die radiale Umfangsfläche in den in Umfangsrichtung zwischen den Befestigungsabschnitten liegenden Bereichen Befestigungselementabschnitte ausbildet, die jeweils zumindest ein Befestigungselement aufweisen.

Demnach ist die radiale Umfangsfläche des Kupplungselements in Umfangrichtung in zumindest zwei Befestigungsabschnitte und zumindest zwei Befestigungselementabschnitte unterteilt. Durch die zumindest zwei Befestigungsabschnitte bzw. Befestigungselementabschnitte kann eine kippsichere Befestigungselementaufnahme bzw. ein kippsicherer Befestigungselementeingriff umgesetzt werden. Wird die Anordnung an einer äußeren Umfangsfläche vorgesehen, ergibt sich hieraus eine gute Zugänglichkeit oder aber auch eine visuelle Kontrollmöglichkeit der Befestigung.

Die vorstehende Ausgestaltung unterstützt zudem die Ausbildung komplementärer oder baugleicher Kupplungssysteme, wie diese später noch adressiert werden, da sich das Kupplungselement sowohl zur Befestigungselementaufnahme als auch zum Befestigungselementeingriff eignet.

In einer Weiterbildung werden die Befestigungsabschnitte durch die Dichtelementaufnahme gebildet, die mittels Freisparungen im Kupplungsgehäuse, die zur Kupplungsseite hin offen sind, von der Kupplungsseite aus zugänglich sind, wobei insbesondere die Befestigungselementabschnitte zu den Befestigungsabschnitten korrespondieren.

Durch die Bildung der Befestigungsabschnitte durch die Dichtelementaufnahme kann gewährleistet werden, dass die Dichtelementaufnahme und somit das kupplungsseitenseitige Dichtelement bei einer Befestigung mit einem weiteren Kupplungselement oder Aufsatz zu diesem in positionsfester Beziehung gehalten wird. Über die Freisparungen im Kupplungsgehäuse, die zur Kupplungsseite hin offen sind, wird eine gute Zugänglichkeit der Befestigungsabschnitte erreicht.

Im Sinne der Verwendung komplementärer oder baugleicher Kupplungselemente für eine Kupplungssystem bzw. komplementärer Kupplungselemente und Aufsätze ist es darüber hinaus von Vorteil, wenn die Befestigungselementabschnitte zu den Befestigungsabschnitten insofern korrespondieren, dass die Befestigungsabschnitte bzw. die hierfür vorgesehenen Freisparungen zur Konnektierung die Befestigungselementabschnitte führen. Entsprechend sollte die Breite der Befestigungsabschnitte bzw. der Freisparungen in Umfangsrichtung im Wesentlichen der Breite der Befestigungselementabschnitte bzw. der Bereiche zwischen den Freisparungen entsprechen. Der Begriff "im Wesentlichen" ist darauf gerichtet, dass zur ungehinderten Führung ein vorbestimmtes Spiel vorgesehen werden kann und/oder fertigungsbedingte Toleranzen Einfluss nehmen können. Ebenso können bestimmte Formgebungen zu Abweichungen führen, die aber keinen Einfluss auf die überwiegenden Führungseigenschaften haben sollten.

Insbesondere wird das zumindest eine Befestigungselement des jeweiligen Befestigungselementabschnitts durch die Dichtelementaufnahme gebildet.

Vergleichbar zur Bildung der Befestigungsabschnitte durch die Dichtelementaufnahme kann auch die Bildung der Befestigungselemente durch die Dichtelementaufnahme die positionsfeste Befestigung der Dichtelementaufnahme unterstützen. Bei komplementären oder baugleichen Kupplungselementen werden beispielsweise insbesondere bei Bildung der Befestigungsabschnitte und der Befestigungselemente durch die Dichtelementaufnahme die Dichtelementaufnahmen der Kupplungselemente im konnektierten Zustand positionssicherzueinander gehalten. Dadurch kann eine vorbestimmte Flächenpressung zwischen den jeweils kupplungsseitenseitigen Dichtflächen gewährleistet werden, um eine fluiddichte Verbindung der Kupplungselemente zu sichern, so dass ein Fluidaustausch erfolgen kann, ohne dass das auszutauschende Fluid nach außen austreten kann.

Das zumindest eine Befestigungselement kann beispielsweise als sich seitlich in Richtung der Kupplungsseite über das kupplungsseitenseitige Dichtelement hinausragendes Schnapphakenelement ausgebildet sein.

In einer Weiterbildung sind die Befestigungselemente der Dichtelementaufnahme über das Kupplungsgehäuse in einem Befestigungsabschnitt fixierbar.

Das Kupplungsgehäuse umgibt hierzu beispielsweise die Befestigungselemente der Dichtelementaufnahme im Bereich der Befestigungselementabschnitte. Mit anderen Worten bildet das Gehäuse im Bereich der Befestigungselementabschnitte jeweils einen in Richtung der Kupplungsseite vorragenden Kupplungsgehäuseabschnitt aus. Das Dichtelement und das Kupplungsgehäuse sind dabei in einer Richtung entlang des Dorns relativ zueinander bewegbar, so dass auch das Befestigungselement in einer Richtung entlang des Dorns relativ zum Kupplungsgehäuse bewegbar ist. Das Kupplungsgehäuse ist dabei derart ausgebildet, dass das Befestigungselement in einem dekonnektierten Zustand mit einem Befestigungsabschnitt eines weiteren Kupplungselements oder Aufsatzes in Überdeckung gebracht werden kann. Wird nun das Kupplungselement in einen konnektierten Zustand durch Bewegung der Dichtelementaufnahme von einer der Kupplungsseite zugewandten Bewegungsendposition in eine dem Dorn zugewandte Bewegungsendposition überführt, schiebt sich das Kupplungsgehäuse bzw. ein durch dieses gebildeter vorragende Kupplungsgehäuseabschnitt weiter über das Befestigungselement, wobei die mit dem Befestigungselement zusammenwirkenden Kontur des Kupplungsgehäuses das Befestigungselement in eine Position bewegt, in der es mit dem Befestigungsabschnitt des weiteren Kupplungselements oder Aufsatzes in Eingriff gebracht und gehalten wird. In einer beispielhaften Ausgestaltung ist das jeweilige Befestigungselement als Schnapphakenelement ausgebildet, wobei insbesondere der den Schnapphaken aufweisende Abschnitt des Befestigungselements einen radial nach außen weisenden Vorsprung aufweist. Dieser Vorsprung liegt im dekonnektierten Zustand auf einem durch das Kupplungsgehäuse auf seiner Innenseite gebildeten radialen Absatz auf. Wird nun die Dichtelelementaufnahme in Richtung der Dornaufnahme bewegt, wird der Vorsprung am Absatz vorbeigeführt und gelangt in einen Bereich mit kleinerem Innendurchmesser. Folglich wird das Befestigungselement in diesem Bereich radial nach innen gedrückt und wird im Eingriff mit einem korrespondierenden Befestigungsabschnitt gehalten. Die Verbindung kann somit erst wieder im dekonnektierten Zustand gelöst werden.

In einer Ausgestaltung sind die Befestigungsabschnitte und die Befestigungselementabschnitte komplementär zueinander ausgebildet, so dass bei einer Verbindung mit einem komplementären oder baugleichen Kupplungselement eine fluiddichte Verbindung geschaffen wird.

Die komplementäre Ausbildung der Befestigungsabschnitte und Befestigungselementabschnitte betrifft eine Ausgestaltung, bei der die Befestigungsabschnitte und Befestigungselementabschnitte zueinander korrespondieren. Dies kann durch die Verwendung baugleicher Kupplungselemente für ein Kupplungssystem verdeutlicht werden, bei dem die Befestigungsabschnitte des einen Kupplungselements zu den Befestigungselementabschnitten des anderen Kupplungselements korrespondieren und umgekehrt. Bei komplementären Kupplungselementen ist es dabei zumindest ausreichend, dass die Befestigungsabschnitte und Befestigungselementabschnitte zueinander korrespondieren. Mit anderen Worten ist der Befestigungsabschnitt eine Projektion bzw. ein Negativ seines jeweiligen Befestigungselementabschnitts und umgekehrt.

In einer weiteren Ausgestaltung ist das Kupplungsgehäuse gegenüber der Dichtelementaufnahme erst dann relativ bewegbar, wenn das jeweilige Befestigungselement bei Kopplung mit einem komplementären oder baugleichen Kupplungselement mit dem Befestigungsabschnitt des anderen Kupplungselements einen Kupplungszustand ausbildet.

Im Falle des vorstehend exemplarisch beschriebenen Schnapphakens mit radialem Vorsprung als Befestigungselement kann der Schnapphaken beispielsweise den durch das Kupplungsgehäuse gebildeten Absatz nur dann überwinden, wenn der Schnapphaken zumindest gemäß der radialen Erstreckung des Absatzes nach innen bewegt werden kann. Dies kann insbesondere zunächst dadurch verhindert werden, dass das zu koppelnde Kupplungselement bis zum Erreichen des Eingriffs für das Befestigungselement im Befestigungsabschnitt im Bereich der Schnapphaken einen Durchmesser aufweist, der der die erforderliche Radialbewegung des Schnapphakens nicht zulässt. Erst der durch eine Aussparung gebildete Eingriff ermöglicht dann die Bewegungsfreigabe. Im Sinne komplementärer oder baugleicher Kupplungselemente kann das Erfordernis in Bezug auf zwei Kupplungselemente auf ein Kupplungselement projiziert werden.

Die Erfindung ist auch auf einen Aufsatz für ein vorstehend beschriebenes Kupplungselement gerichtet, wobei der Aufsatz einen Befestigungsabschnitt umfasst, der so ausgebildet ist, dass er in einer der Dornaufnahme zugewandten Bewegungsendposition der Dichtelementaufnahme im Kupplungselement fixierbar ist.

Der Aufsatz kann beispielsweise für die Verbindung mit einer Spritze oder anderen Einrichtungen eines Fluidtransfersystems vorgesehen werden. Entsprechend kann über den Aufsatz das Anwendungsgebiet eines vorstehenden beschriebenen Kupplungselements erweitert werden. Durch den erfindungsgemäß ausgebildeten Befestigungsabschnitt kann eine Konnektierung zum Fluidaustausch vergleichbar zu einer Kopplung zweier vorstehend beschriebener Kupplungselemente erfolgen, so dass für den Aufsatz kupplungselementseitig keine weiteren Vorkehrungen getroffen werden müssen.

In einem weiteren Aspekt betrifft die Erfindung ein Kupplungssystem mit zwei vorstehend beschriebenen Kupplungselementen.

Das Kupplungssystem weist entsprechend die zu den Kupplungselementen beschriebenen Vorteile auf.

In einer Ausgestaltung sind die Kupplungselemente komplementär oder baugleich und insbesondere durch einen relativen Winkelversatz um ihre Längsachse gemäß der Positionen der Befestigungsabschnitte, bevorzugt um 90°, koppelbar.

Durch die komplementäre oder baugleiche Ausgestaltung kann Variantenvielzahl verringert werden, so dass neben Vorteilen in der Vorhaltung entsprechender Kupplungselemente für ein Kupplungssystem auch eine Verwechselung von Kupplungselemente vermieden werden kann. Im Sinne komplementärer Kupplungselemente können zudem wiederum verschiedene Varianten vorgesehen werden, ohne dass dies Einfluss auf die Kupplung der Kupplungselemente selbst haben muss.

In einer Weiterbildung ist zumindest eine der jeweiligen vorderen Fluidöffnungen, insbesondere beide vorderen Fluidöffnungen, in einem Kupplungszustand, in dem sich die jeweiligen Dichtelementaufnahmen in einer zu der jeweiligen Dornaufnahme zugewandten Bewegungsendposition befinden, in der fluiddichten Kammer des jeweils anderen Kupplungselements angeordnet und steht mit der jeweiligen hinteren Fluidöffnung des anderen Kupplungselements, das vorzugsweise im Kupplungszustand ebenfalls in dieser fluiddichten Kammer angeordnet ist, in fluider Verbindung.

Demnach befindet sich eine vordere Fluidöffnung im konnektierten Zustand in der fluiddichten Kammer, in der auch die hintere Fluidöffnung des konnektierten anderen Kupplungselements angeordnet ist, so dass hierüber ein Fluidaustausch stattfinden kann. Im Sinne komplementärer, insbesondere baugleicher Kupplungselemente ist der Dorn derart ausgebildet, dass das entsprechende Kupplungssystem die Anordnung der jeweils vorderen Fluidöffnungen in der fluiddichten Kammer in Verbindung mit der hinteren Fluidöffnung wechselseitig vorsieht. Ein Fluidaustausch kann somit ausgehend von dem einen wie auch dem anderen Kupplungselement ermöglicht werden.

Insbesondere sind die der Kupplungsseite zugewandten Dichtflächen der Dichtelemente der Kupplungselemente im Kupplungszustand in Kontakt und der Kontakt wird bei einer Entkopplung erst aufgehoben, wenn die jeweiligen Dichtelementaufnahmen in einer der jeweiligen Dornaufnahme abgewandten Bewegungsendposition sind.

Durch den Kontakt der jeweils der Kupplungsseite zugewandten Dichtflächen kann die Verbindung zwischen den Kupplungselementen fluiddicht erfolgen. Insbesondere stehen die Dichtflächen dabei nicht einfach nur in Kontakt, sondern werden unter einer vorbestimmten Flächenpressung gehalten.

Um möglichst erst dann die fluiddichte Verbindung der Kupplungselemente aufzuheben, wenn die jeweils vordere Fluidöffnung nicht mehr über die Dichtfläche in Richtung der Kupplungsseite vorsteht, insbesondere der Dorn mit gegebenenfalls noch darin befindlichen Fluidrückständen insgesamt, kann die Möglichkeit der Lösung der Kontaktflächen beispielsweise erst dann vorgesehen werden, wenn sich die Kupplungselemente im dekonnektierten Zustand, wie dies durch Erreichen der Dichtelementaufnahme in einer der ihr zugeordneten Dornaufnahme abgewandten Bewegungsendposition erfolgen kann, befinden. In einer Umsetzungsausgestaltung umfasst zumindest eines der Kupplungselemente hierzu beispielweise ein Befestigungselement, das durch das Kupplungsgehäuse bis zum Erreichen eines dekonnektierten Zustands oder zumindest bis kurz davor in einer Befestigungsposition arretiert wird.

Die Erfindung betrifft zudem ein Kupplungskit mit einem Aufsatz und einem Kupplungselement gemäß den vorstehenden Ausführungen oder einem vorstehend beschriebenen Kupplungssystem.

Das Kupplungskit weist die zu seinen Komponente beschriebenen Vorteile auf.

Merkmale, Zweckmässigkeiten und Vorteile der Erfindung werden nachfolgend auch anhand von Ausführungsbeispielen unter Bezugnahme auf die Zeichnungen beschrieben.

Es zeigt
Fig. 1 eine perspektivische Ansicht zweier Kupplungselemente gemäß einer ersten Ausführungsform in einem dekonnektieren Zustand, die ein Kupplungssystem nach Fig. 4 bilden;
Fig. 2 eine Querschnittsansicht eines Kupplungselements gemäß Fig. 1 in einer Schnittebene durch die Befestigungsabschnitte;
Fig. 3 eine Querschnittsansicht eines Kupplungselements gemäß Fig. 1 in einer Schnittebene durch die Befestigungselementabschnitte;
Fig. 4 eine Querschnittsansicht eines Kupplungssystems mit zwei (einem Paar) Kupplungselementen gemäß der ersten Ausführungsform in einem konnektierten Zustand;
Fig. 5 eine Querschnittsansicht eines Kupplungselements gemäß einer zweiten Ausführungsform in einer Schnittebene durch die Befestigungsabschnitte;
Fig. 6 eine Querschnittsansicht eines Kupplungselements gemäß der zweiten Ausführungsform in einer Schnittebene durch die Befestigungselementabschnitte;
Fig. 7 eine Querschnittsansicht eines Kupplungssystems mit zwei (einem Paar) Kupplungselementen gemäß der zweiten Ausführungsform in einem konnektierten Zustand;
Fig. 8 eine Querschnittsansicht eines Kupplungselements gemäß einer dritten Ausführungsform in einer Schnittebene durch die Befestigungsabschnitte;
Fig. 9 eine Querschnittsansicht eines Kupplungselements gemäß der dritten Ausführungsform in einer Schnittebene durch die Befestigungselementabschnitte;
Fig. 10 eine Querschnittsansicht eines Kupplungssystems mit zwei (einem Paar) Kupplungselementen gemäß der dritten Ausführungsform in einem konnektierten Zustand;
Fig. 11 eine Querschnittsansicht eines Kupplungselements gemäß der dritten Ausführungsform in einer Schnittebene durch die Befestigungselementabschnitte sowie einen Aufsatz in einem dekonnektieren Zustand gemäß eines Kupplungskits nach Fig. 12;
Fig. 12 eine Querschnittsansicht eines Kupplungskits mit einem Kupplungselement gemäß der dritten Ausführungsform und einem Aufsatz in einem konnektierten Zustand;
Fig. 13 eine Übersicht der Außenansichten des Kupplungssystems gemäß den Figuren 1 bis 4, die Schnittansicht entlang der Schnittlinie A-A sowie eine perspektivische Ansicht im dekonnektierten Zustand;
Fig. 14 eine Übersicht der Außenansichten des Kupplungssystems gemäß den Figuren 1 bis 4 bzw. Fig. 13, die Schnittansicht entlang der Schnittlinie A-A sowie eine perspektivische Ansicht im konnektierten Zustand;
Fig. 15 eine Übersicht der Außenansichten des Kupplungssystems gemäß den Figuren 5 bis 7, die Schnittansicht entlang der Schnittlinie A-A sowie eine perspektivische Ansicht im dekonnektierten Zustand;
Fig. 16 eine Übersicht der Außenansichten des Kupplungssystems gemäß den Figuren 5 bis 7 bzw. Fig. 15, die Schnittansicht entlang der Schnittlinie A-A sowie eine perspektivische Ansicht im konnektierten Zustand;
Fig. 17 eine Übersicht der Außenansichten des Kupplungssystems gemäß den Figuren 8 bis 10, die Schnittansicht entlang der Schnittlinie A-A sowie eine perspektivische Ansicht im dekonnektierten Zustand;
Fig. 18 eine Übersicht der Außenansichten des Kupplungssystems gemäß den Figuren 8 bis 10 bzw. Fig. 17, die Schnittansicht entlang der Schnittlinie A-A sowie eine perspektivische Ansicht im konnektierten Zustand;
Fig. 19 eine Übersicht der Außenansichten des Kupplungssystems gemäß den Figuren 11 und 12, die Schnittansicht entlang der Schnittlinie A-A sowie eine perspektivische Ansicht im dekonnektierten Zustand;
Fig. 20 eine Übersicht der Außenansichten des Kupplungssystems gemäß den Figuren 11 und 12 bzw. Fig. 19, die Schnittansicht entlang der Schnittlinie A-A sowie eine perspektivische Ansicht im konnektierten Zustand.

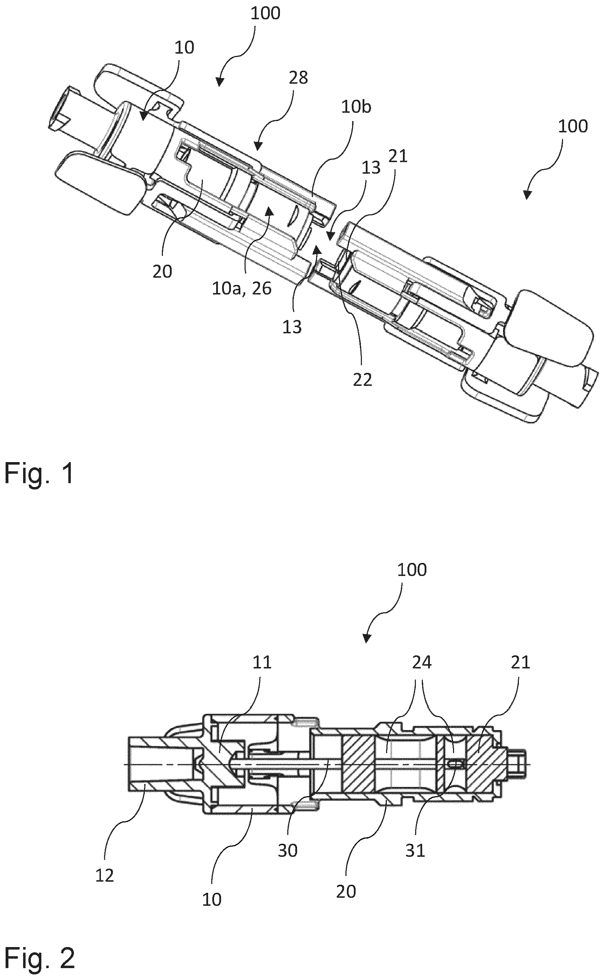

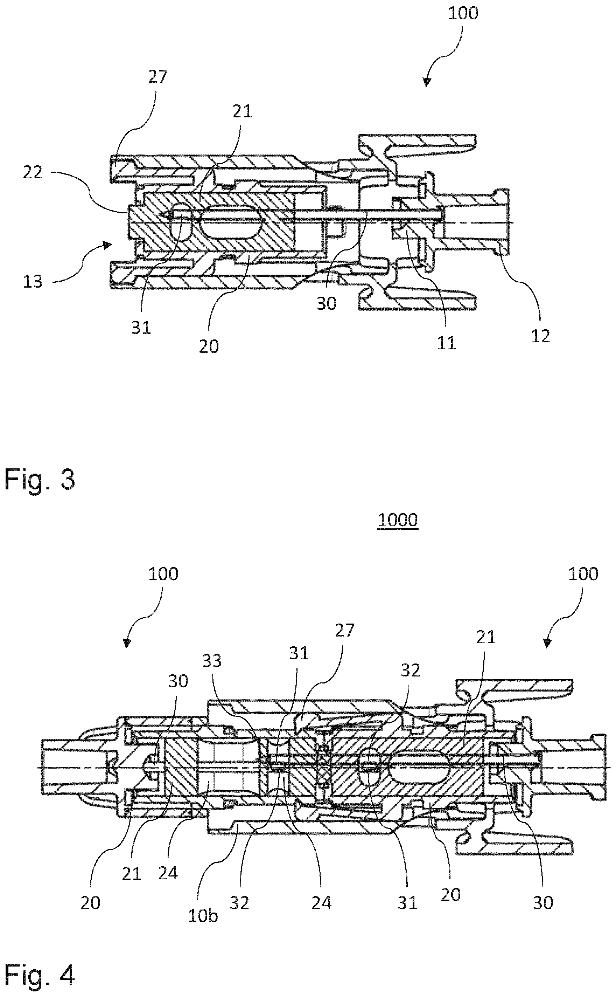

Fig. 1 zeigt eine perspektivische Ansicht zweier Kupplungselemente 100 gemäß einer ersten Ausführungsform in einem dekonnektieren Zustand, die ein Kupplungssystem 1000 nach Fig. 4 bilden. Die Kupplungselemente 100 umfassen jeweils ein Kupplungsgehäuse 10, das sich entlang einer Längsachse von einem Fluidanschluss 12 zu einer Kupplungsseite 13 erstreckt. Das Kupplungsgehäuse 10 weist zudem vorzugsweise zwei einander gegenüberliegende und zur Kupplungsseite 13 hin offene Freisparungen 10a auf, die zwischen zur Kupplungsseite 13 vorragenden Kupplungsgehäuseabschnitten 10b gebildet werden. Im Kupplungsgehäuse 10 ist eine Dichtelementaufnahme 20 angeordnet, die in dieser Ausführungsform von einer der Kupplungsseite 13 zugewandten Bewegungsendposition in eine der Dornaufnahme 11, wie sie beispielsweise in Fig. 2 mit dem Dorn 30 gezeigt ist, zugewandten Bewegungsendposition und umgekehrt entlang des Dorns 30 bewegbar ist. In dem hier dargestellten dekonnektierten Zustand befindet sich die Dichtelementaufnahme 20 in ihrer der Kupplungsseite 13 zugewandten Bewegungsendposition. Die Dichtelementaufnahme 20 bildet im Bereich der Freisparungen 10a jeweils einen Befestigungsabschnitt 26 aus, der dem Eingriff einen Befestigungselements 27, wie beispielsweise in Fig. 3 gezeigt, dient. Hierzu weist der Befestigungsabschnitt 26 in der ersten Ausführungsform eine sich in Umfangsrichtung erstreckende Nut auf. Zwischen den Befestigungsabschnitten 26 liegen Befestigungselementabschnitte 28, die jeweils zumindest ein Befestigungselement 27, wie dies in Bezug auf Fig. 3 gezeigt und weiter erläutert wird, aufweisen. Zudem ist in Fig. 1 eine der Kupplungsseite 13 zugewandte Dichtfläche 22 eines Dichtelements 21, wie auch in Fig. 2 dargestellt, angedeutet. Zumindest der die Dichtfläche 22 aufweisende Dichtelementabschnitt ist positionsfest mit der Dichtelementaufnahme 20 verbunden, um die Dichtelementaufnahme 20 fluiddicht abzudichten. Im dekonnektierten Zustand ragt der Dorn 30 nicht in Richtung der Kupplungsseite über die Dichtfläche 22 hinaus.

Zur weiteren Veranschaulichung der Ausgestaltung des Kupplungselements 100 gemäß der ersten Ausführungsform zeigt Fig. 2 eine Querschnittsansicht des Kupplungselements 100 gemäß Fig. 1 in einer Schnittebene durch die Befestigungsabschnitte 26 der Dichtelementaufnahme 20 im dekonnektierten Zustand. Das Dichtelement 21 weist zwischen seiner der Kupplungsseite 13 zugewandten Dichtfläche 22 und seinem der Dornaufnahme 11 zugewandten Seite zwei fluiddichte Kammern 24 auf. Die der Kupplungsseite 13 zugewandte fluiddichte Kammer 24 ist im dekonnektierten Zustand um die vordere fluidöffnung 31 des Dorns 30 angeordnet. Die hintere Fluidöffnung 32, die hier in Bezug auf die Längsachse des Dorns 30 einen Winkelversatz zur vorderen Fluidöffnung 31 aufweist und somit in Fig. 2 nicht erkennbar ist, ist in der der Kupplungsseite 13 abgewandten fluiddichten Kammer 24 angeordnet. Durch die Ausgestaltung und Anordnung des Dichtelements 21 in Zusammenwirken mit den Fluidöffnungen 31, 32 ist die Dichtelementaufnahme 20 in Bezug auf die Fluidöffnungen 31, 32 fluiddicht, so dass beispielsweise auf eine weitere Abdichtung zwischen dem Kupplungsgehäuse 10 und der Dichtelementaufnahme 20 verzichtet werden kann. Das Dichtelement 21 ist somit hier auch insgesamt positionsfest mit der Dichtelementaufnahme 20 verbunden und wird über die Bewegung der Dichtelementaufnahme 20 gleichsam relativ entlang des Dorns 30 bewegt.

Wie aus Fig. 3 ersichtlich, die eine Querschnittsansicht eines Kupplungselements 100 gemäß Fig. 1 in einer Schnittebene durch die Befestigungselementabschnitte 28 zeigt, umfassen die sich genüberliegenden Befestigungselementabschnitt 28 jeweils ein Befestigungselement, das hier exemplarisch aus einem sich seitlich in Richtung der Kupplungsseite 13 erstreckenden Schnapphakenelement gebildet wird. Um die Schnapphaken der Befestigungselemente 27 in einer Nut eines Befestigungsabschnitts 26, wie bei einer Verbindung baugleicher Kupplungselemente 100, arretieren zu können, weisen die Schnapphaken jeweils einen radial nach außen weisenden Vorsprung auf, der im dekonnketierten Zustand in einer radialen Aussparung in der vorragenden Kupplungsgehäuseabschnitten 10b aufliegt. Ausgehend von der kupplungsseitenseitigen Aussparung erstreckt sich die Führungsstrecke des Kupplungsgehäuseabschnitts 10b zur Führung des Schnapphakens des Befestigungselements 27 mit kleinerem Durchmesser gegenüber der Aussparung in Richtung der Dornaufnahme 11.Wird der Schnapphaken des Befestigungselements 27 in Richtung der Dornaufnahme bewegt und an der Aussparung vorbei in den Bereich mit kleinerem Innendurchmesser geführt, so werden die Schnapphaken radial nach innen gedrückt und können somit mit einem Befestigungsabschnitt 26 eines weiteren Kupplungselements 100 in Eingriff gebracht und arretiert werden. Die Arretierung kann erst wieder in einem dekonnektierten Zustand gelöst werden, in dem die Vorsprünge wieder radial in die Aussparungen zurückbewegbar sind.

Zur Veranschaulichung einer exemplarischen Ausgestaltung eines erfindungsgemäßen Kupplungssystems und zur weiteren Erläuterung des Kupplungselements 100 zeigt Fig. 4 eine Querschnittsansicht eines Kupplungssystems 1000 mit zwei Kupplungselementen 100 gemäß der ersten Ausführungsform in einem konnektierten Zustand. Zur Konnektierung werden die baugleichen Kupplungselemente 100 mit ihren Kupplungsseiten einander gegenüberliegend um 90° versetzt zueinander angeordnet und ineinander geschoben. Die vorragenden Kupplungsgehäuseabschnitte 10b des jeweils einen Kupplungselements 100 werden dabei in die zu Ihnen korrespondierenden Freisparungen 10 des jeweils anderen Kupplungselements 100 bewegt. Im Bereich der Freisparungen weist die Dichtelementaufnahme 20 dabei einen Durchmesser auf, durch den die Schnapphaken der Befestigungselemente 27 zunächst in den Aussparungen der vorragenden Kupplungsgehäuseabschnitte 10b gehalten werden. mit Erreichen der jeweiligen Nuten der Befestigungsabschnitte 26 des jeweils anderen Kupplungselements 100 können die Schnapphaken der Befestigungselemente 27 in diese eingreifen, so dass die Schnapphaken aus der jeweiligen Aussparung radial nach innen gedrückt werden können. Dadurch kann die Dichtelementaufnahme 20 nun aus ihrer der Kupplungsseite 13 zugewandten Bewegungsendposition in Richtung der Dornaufnahme 11 bis zum Erreichen der der Dornaufnahme 11 zugewandten Bewegungsendposition und damit bis zum Erreichen des konnektierten Zustand entlang des Dorns bewegt werden. Durch den kleineren Innendurchmesser entlang dieser Führungsstrecke der Schnapphaken gegenüber dem Durchmesser der Aussparungen werden die Schnapphaken im Eingriff mit den Nuten arretiert. Die Arretierung hält dabei die jeweiligen kupplungsseitenseitigen Dichtflächen 22 der Kupplungselemente 100 mit vorbestimmter Flächenpressung und dadurch fluiddicht zusammen.

Im konnektierten Zustand ragt nun einerseits die vordere Fluidöffnung 31 des jeweiligen Kupplungselements 100 über ihre jeweilige kupplungsseitenseitige Dichtfläche 22 hinaus vor, durchläuft den ersten Abschnitt des Dichtelements 20 des jeweils anderen Kupplungselements 100 und ist in diesem Dichtelement 20 in der der Kupplungsseite 13 zugewandten fluiddichten Kammer 24 angeordnet. Andererseits schieben sich die jeweiligen der Kupplungsseite 13 zugewandten fluiddichten Kammern 24 der jeweiligen Kupplungselemente 100 über die jeweiligen hinteren Fluidöffnungen 32. Folglich sind somit im konnektierten Zustand in den jeweils der Kupplungsseite zugewandten fluiddichten Kammern 24 sowohl die hintere Fluidöffnung 32 des jeweiligen Kupplungselements 100 als auch die vordere Fluidöffnung 31 des jeweils anderen Kupplungselements 100 angeordnet, so dass ein Fluidaustausch beiderseitig möglich ist.

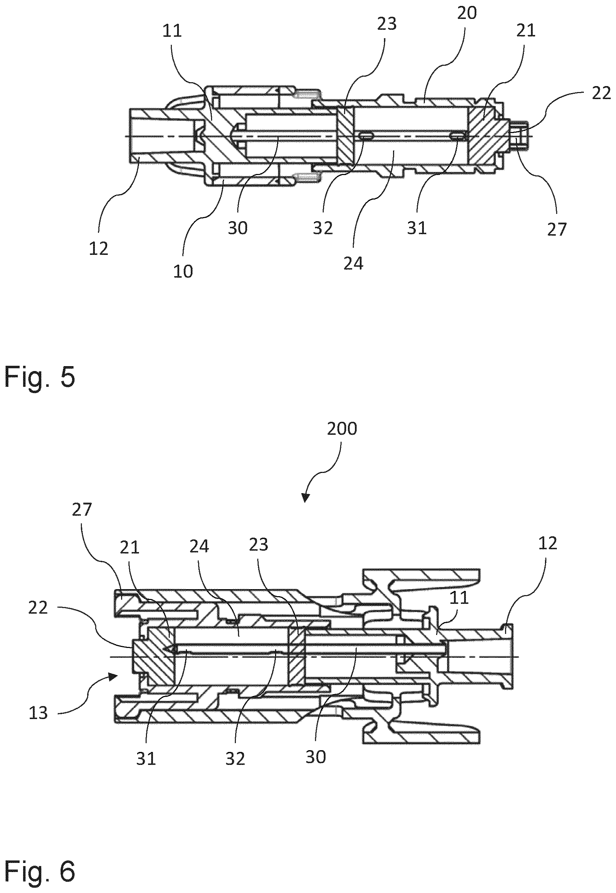

Fig. 5 zeigt eine Querschnittsansicht eines Kupplungselements 200 gemäß einer zweiten Ausführungsform in einer Schnittebene durch die Befestigungsabschnitte 26 und Fig. 6 eine Querschnittsansicht des Kupplungselements 200 gemäß der zweiten Ausführungsform in einer Schnittebene durch die Befestigungselementabschnitte 28. In Anlehnung an die erste Ausführungsform werden für gleiche Komponenten gleiche Bezugszeichen verwendet, auch wenn konkrete Ausgestaltungsmerkmale voneinander abweichen können.

Das Kupplungselement 200 gemäß der zweiten Ausführungsform unterscheidet sich von dem Kupplungselement 100 der ersten Ausführungsform im Wesentlichen dadurch, dass das Kupplungselement 200 nur eine fluiddichte Kammer 24 aufweist. Die fluiddichte Kammer 24 wird auch nicht integral durch ein Dichtelement 21 ausgebildet, sondern durch ein der Kupplungsseite 13 zugewandtes Dichtelement 21 und ein der Dornaufnahme 11 zugewandtes Dichtelement 23. Das der Kupplungsseite 13 zugewandte Dichtelement 21 ist positionsfest mit der Dichtelementaufnahme 20 verbunden, während das der Dornaufnahme 11 zugewandte Dichtelement 23 positionsfest mit dem Dorn verbunden ist. Im dekonnektierten Zustand liegen sowohl die vordere Fluidöffnung 31 als auch die hindere Fluidöffnung 32 in der durch die Dichtelemente 21, 23 gebildeten fluiddichten Kammer 24.

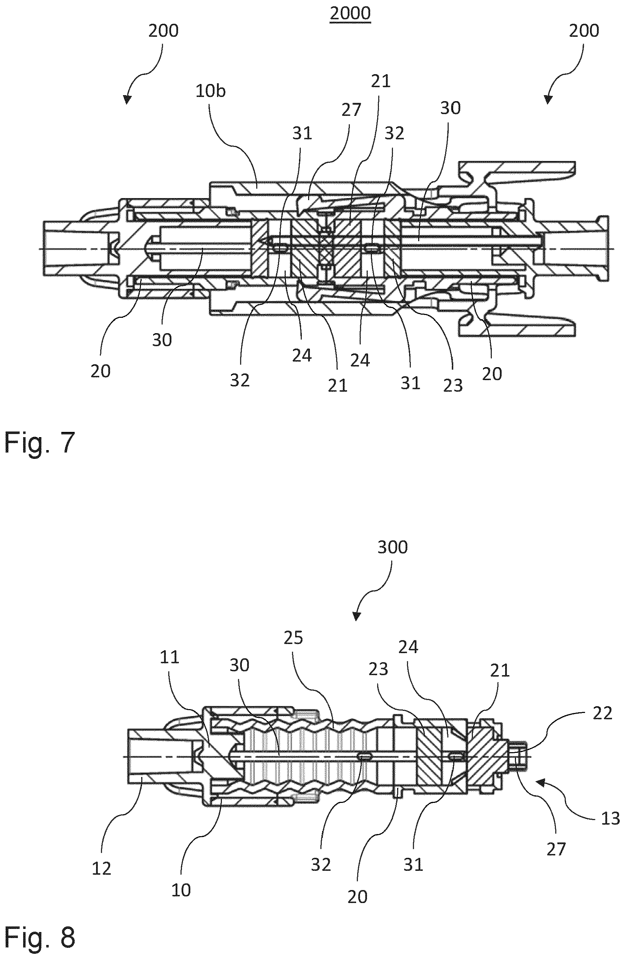

Zur weiteren Veranschaulichung des Kupplungselements 200 zeigt Fig. 7 eine Querschnittsansicht eines Kupplungssystems 2000 mit zwei Kupplungselementen 200 in einem konnektierten Zustand. Gemäß der Bewegung der Dichtelementaufnahmen 20 werden die damit positionsfest verbundenen der Kupplungsseite 13 zugewandten Dichtelemente 21 jeweils in Richtung der dem jeweiligen Kupplungselement 200 zuordenbaren Dornaufnahmen 11 bewegt. In der der jeweiligen Dornaufnahme 11 zugewandten Bewegungsendposition der jeweiligen Dichtelementaufnahme 21 im konnektierten Zustand hat das der Kupplungsseite zugewandte Dichtelement 21 eine Position erreicht, in der die vordere Fluidöffnung 32 über das Dichtelement 21 kupplungsseitenseitig hinausragt und das Dichtelement 21 mit dem Dichtelement 23 weiterhin eine fluiddichte Kammer 24 für die hintere Fluidöffnung 32 ausbildet, wobei die vordere Fluidöffnung des jeweils anderen Kupplungselements im konnektierten Zustand ebenfalls in dieser fluiddichten Kammer 24 angeordnet ist. Mit anderen Worten verkleinert sich das Volumen der durch die Dichtelemente 21, 23 gebildeten fluiddichten Kammer 24 im konnektierten Zustand gegenüber einem dekonnektierten Zustand.

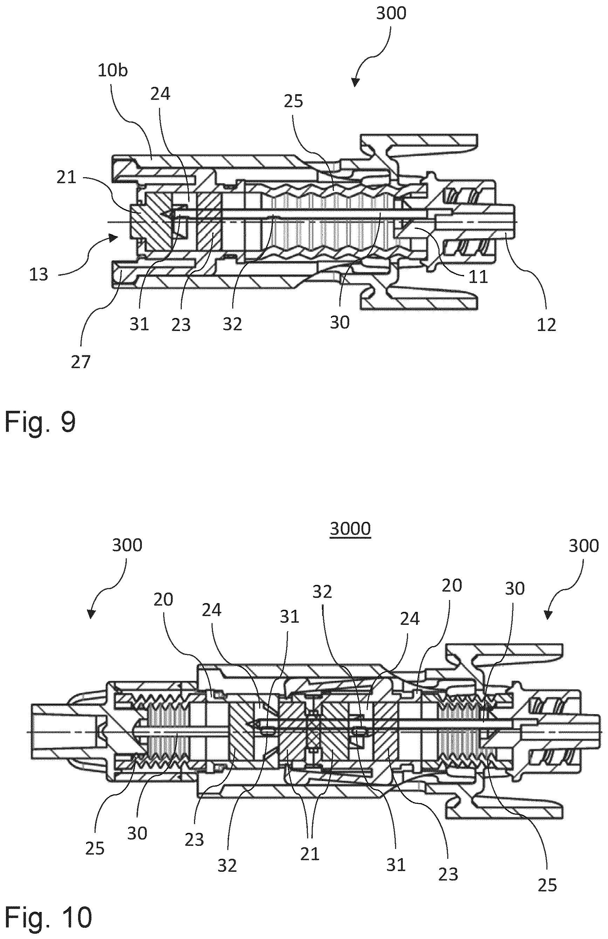

Als weitere Variante zeigt Fig. 8 eine Querschnittsansicht eines Kupplungselements 300 gemäß einer dritten Ausführungsform in einer Schnittebene durch die Befestigungsabschnitte 26 und Fig. 9 eine Querschnittsansicht des Kupplungselements 300 gemäß der dritten Ausführungsform in einer Schnittebene durch die Befestigungselementabschnitte 28. Auch hier werden für vergleichbare Komponenten gleiche Bezugszeichen verwendet.

Wie auch schon das Kupplungselement 200 der zweiten Ausführungsform weist das Kupplungselement 300 der dritten Ausführungsform zwei Dichtelemente 21, 23 auf, wobei hier beide Dichtelemente 21, 23 positionsfest mit der Dichtelementaufnahme 21 verbunden, als entlang des Dorns 30 bewegbar sind. Im dekonnektierten Zustand ist die vordere Fluidöffnung 31 in der durch die Dichtelemente 21, 23 gebildeten fluiddichten Kammer 24 angeordnet, während sich die hintere Fluidöffnung auf einer der Dornaufnahme 11 zugewandten Seite des der Dornaufnahme 11 zugewandten Dichtelements 23 befindet. Um in dieser Konfiguration einen Fluidaustritt, beispielsweise bedingt durch Fluidrückstände an der hinteren Fluidöffnung 32 oder eine unbeabsichtigte oder anderweitige Fluideinleitung in den Dorn 30, zu verhindern und gleichzeitig sie Relativbewegung der Dichtelementaufnahme 20 entlang des Dorns zu ermöglichen, weist das Kupplungsgehäuse 10 ein komprimierbares Dichtelement 25 auf, das die Dornaufnahme 11 mit der Dichtelementaufnahme 20 fluiddicht verbindet. Durch eine elastische Ausgestaltung des komprimierbaren Dichtelements 25 oder anderweitige Maßnahmen, durch die das komprimierbare Dichtelement 25 bestrebt ist, aus einem komprimierten Zustand wieder in einen dekomprimierten Ausgangszustand zu gelangen, kann zudem eine automatische Wiedererreichung des dekonnektierten Zustands unterstützt werden, wenn keine äußeren Kräfte hier gezielt entgegenwirken.

Zur weiteren Veranschaulichung des Kupplungselements 300 zeigt Fig. 10 eine Querschnittsansicht eines Kupplungssystems 3000 mit zwei Kupplungselementen 300 in einem konnektierten Zustand. Gemäß der Bewegung der Dichtelementaufnahmen 20 werden die damit positionsfest verbundenen Dichtelemente 21, 23 jeweils in Richtung der dem jeweiligen Kupplungselement 300 zuordenbaren Dornaufnahmen 11 bewegt. Das der jeweiligen Dornaufnahme 11 zugewandte Dichtelement 23 schiebt sich über die jeweilige hintere Fluidöffnung 32. Im konnektierten Zustand ist dann die hintere Fluidöffnung jeweils in der durch die Dichtelemente 21, 23 gebildeten fluiddichten Kammer 24 angeordnet. Mit anderen Worten verändert die fluiddichte Kammer 24 des Kupplungselements 300 nicht ihr Volumen, sondern ihre Position. Die jeweils vordere Fluidöffnung 31 ragt auch hier wieder in die fluiddichte Kammer 24 des jeweils anderen Kupplungselements 300 hinein.

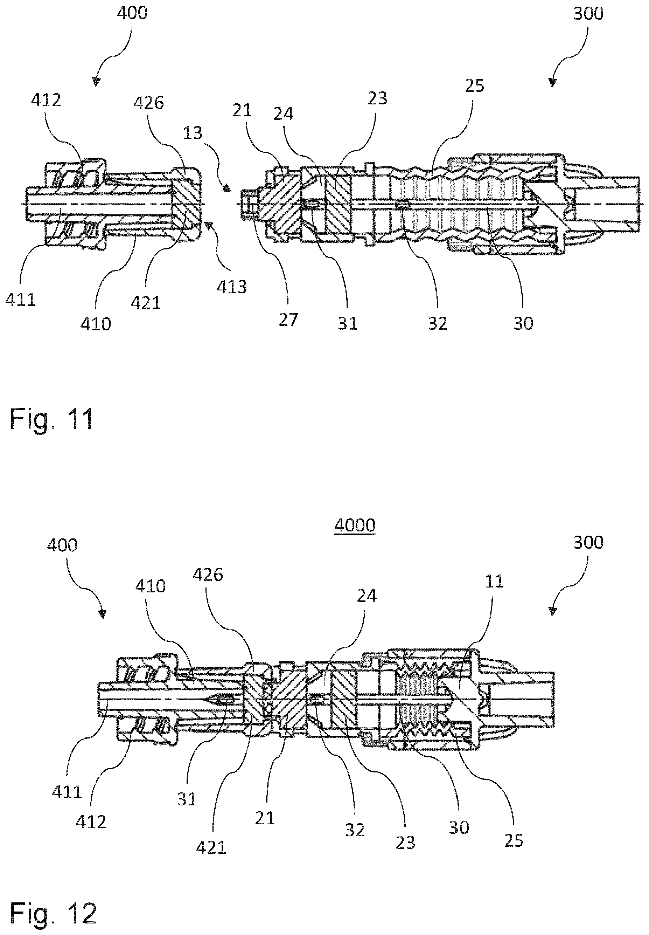

Zur Veranschaulichung eines Aufsatzes in Verbindung mit einem Kupplungselement zeigt Fig. 11 eine Querschnittsansicht des Kupplungselements 300 gemäß der dritten Ausführungsform in einer Schnittebene durch die Befestigungselementabschnitte 28 sowie einen Aufsatz 400 in einem dekonnektieren Zustand gemäß eines Kupplungskits 4000 nach Fig. 12. Der Aufsatz 400 wird durch ein Aufsatzgehäuse 410 gebildet, das einen sich von einem Fluidanschluss 412 in Richtung einer Kupplungsseite 413 erstreckenden Fluidkanal 411 ausbildet. Darüber hinaus umfasst der Aufsatz 400 ein auf der Kupplungsseite 413 durch das Aufsatzgehäuse 410 aufgenommenes Dichtelement 421, das den Fluidkanal 411 auf der Kupplungsseite 413 nach außen abdichtet. Der Aufsatz 400 kann über einen Befestigungsabschnitt 426 mit dem Kupplungselement 300 verbundenen werden. Der Befestigungsabschnitt 426 ist derart konfiguriert, dass er bei Einführung in das Kupplungselement 300 die Schnapphaken der Befestigungselemente 27 in den Aussparungen der vorragenden Kupplungsgehäuseabschnitte 10b hält, die erst mit Erreichen einer vorbestimmten Position der Dichtelemente 421, 21 zueinander zur Arretierung des Befestigungsabschnitts 426 in der Dichtelementaufnahme 21 radial nach innen bewegbar sind. Konkret weist hier der Befestigungsabschnitt 426 exemplarisch über eine sich von der Kupplungsseite 413 in Richtung des Fluidanschlusses 412 erstreckende Länge einen gegenüber dem sich in Richtung des Fluidanschlusses 412 folgenden Aufsatzgehäuseabschnittes größeren Durchmesser auf.

Zur Verbindung des Aufsatzes 400 mit dem Kupplungselement 300 zeigt Fig. 12 eine Querschnittsansicht eines Kupplungskits 4000 mit dem Kupplungselement 300 gemäß der dritten Ausführungsform und dem Aufsatz 400 in einem konnektierten Zustand. Im konnektierten Zustand ist die Dichtelementaufnahme 21 des Kupplungselements 300 in einer der Dornaufnahme 11 zugewandten Bewegungsendposition angeordnet. In dieser Position der Dichtelementaufnahme befindet sich die hintere Fluidöffnung 32 in der fluiddichten Kammer 24 und die vordere Fluidöffnung ragt über die der Kupplungsseite 13 zugewandte Dichtfläche 22 des Dichtelements 21 hinaus vor und ist auf einer dem Fluidanschluss 412 zugewandten Seite des Dichtelements 421 im Fluidkanal 411 angeordnet. In der gezeigten Ausführungsform bildet der Fluidanschluss 412 einen radialen Absatz aus, der zu den radialen Aussparungen in den vorragenden Kupplungsgehäuseabschnitten 10b korrespondiert. Demnach bilden die radialen Aussparungen in den vorragenden Kupplungsgehäuseabschnitten 10b für den Fluidanschluss 412 und somit für das Aufsatzgehäuse 410 einen Anschlag aus, durch denn die Einführung des Aufsatzes 400 in das Kupplungselement 300 begrenzt wird. Hieraus ergibt sich dann auch analog die der Dornaufnahme 11 zugewandte Bewegungsendposition der Dichtelementaufnahme 21.

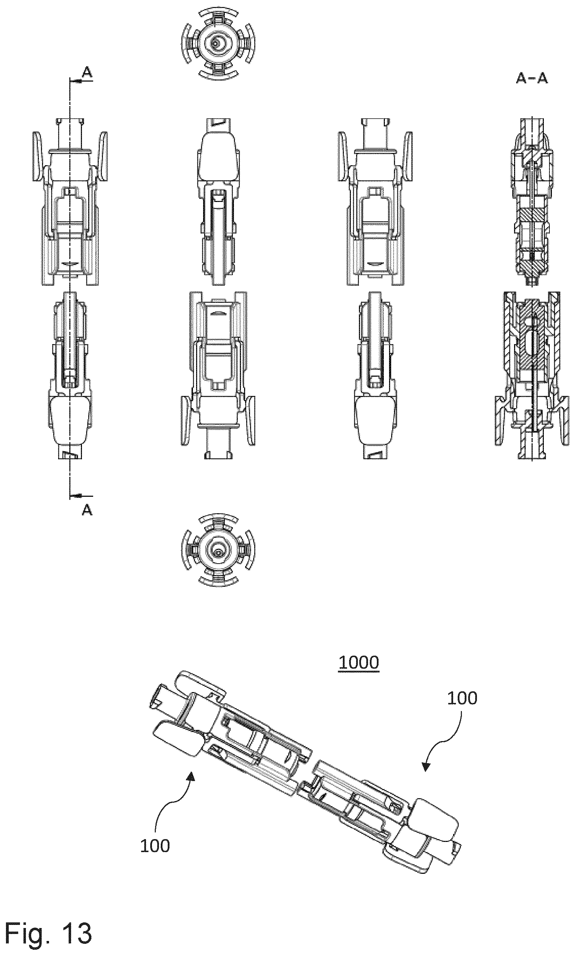

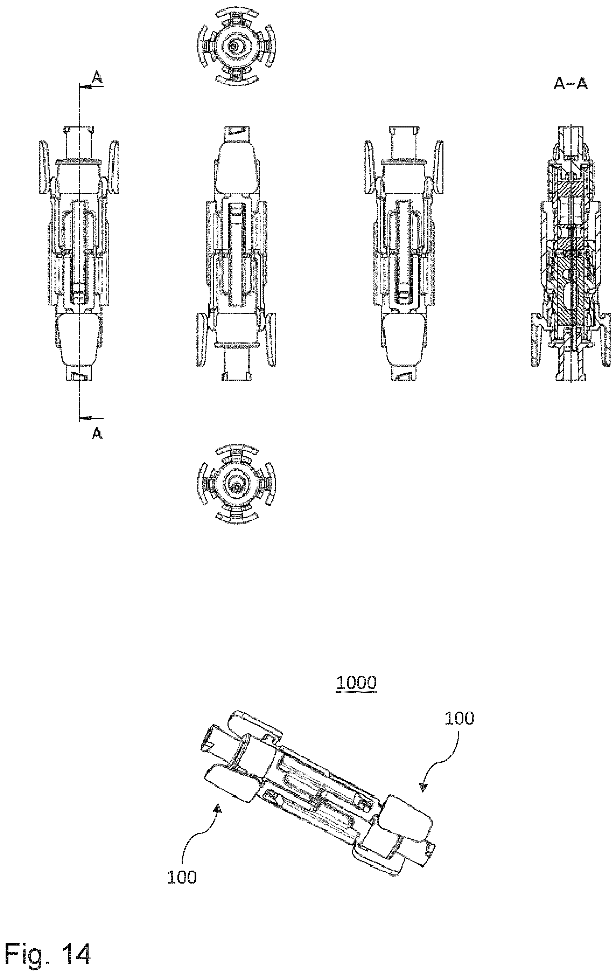

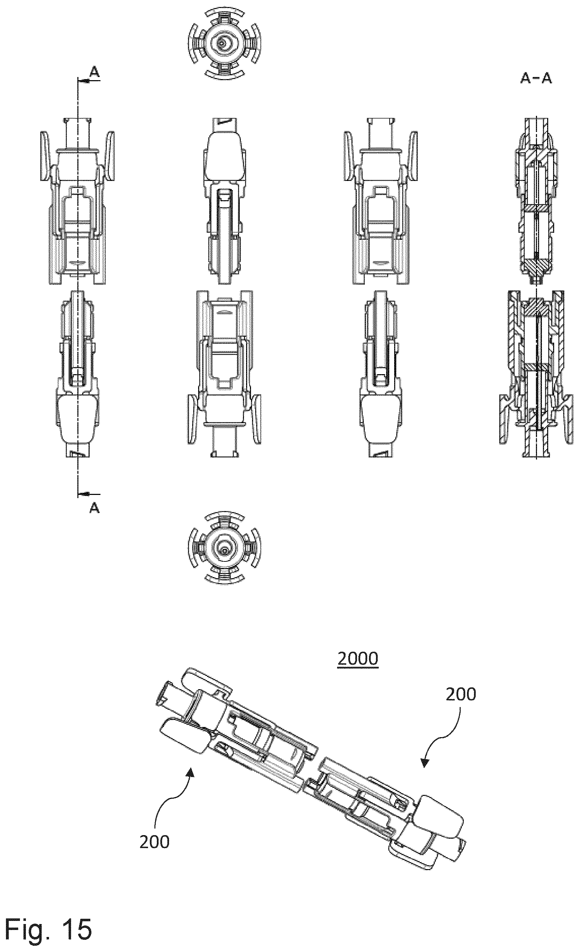

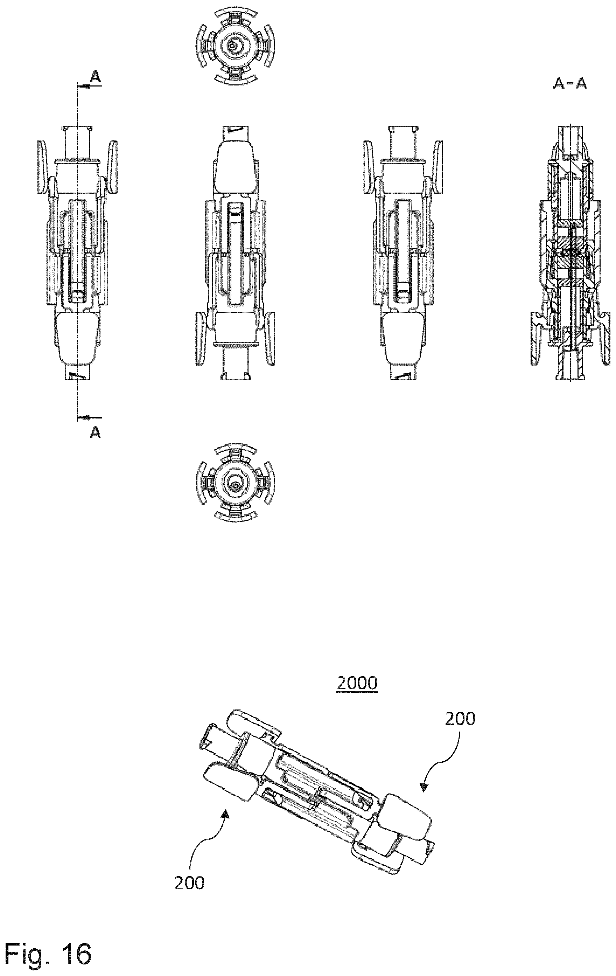

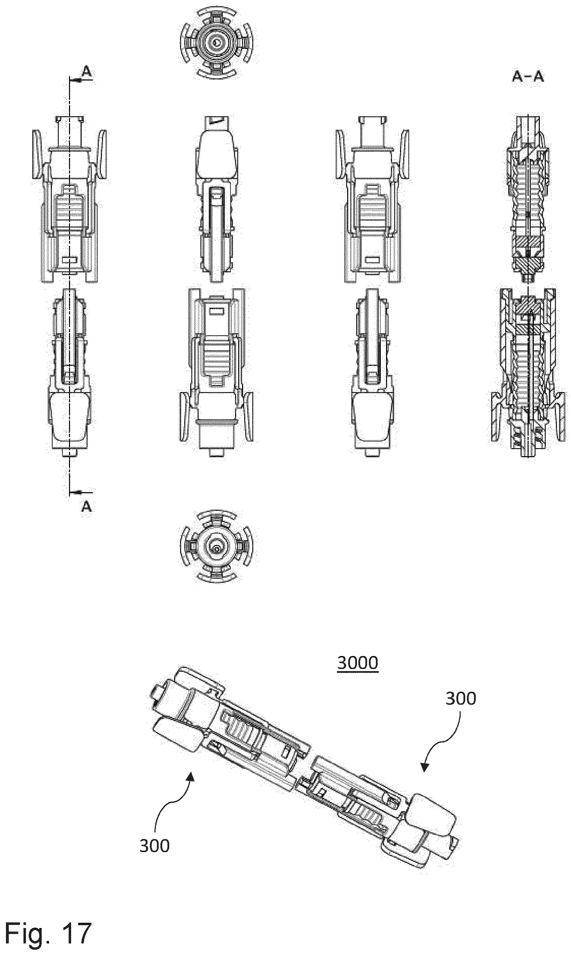

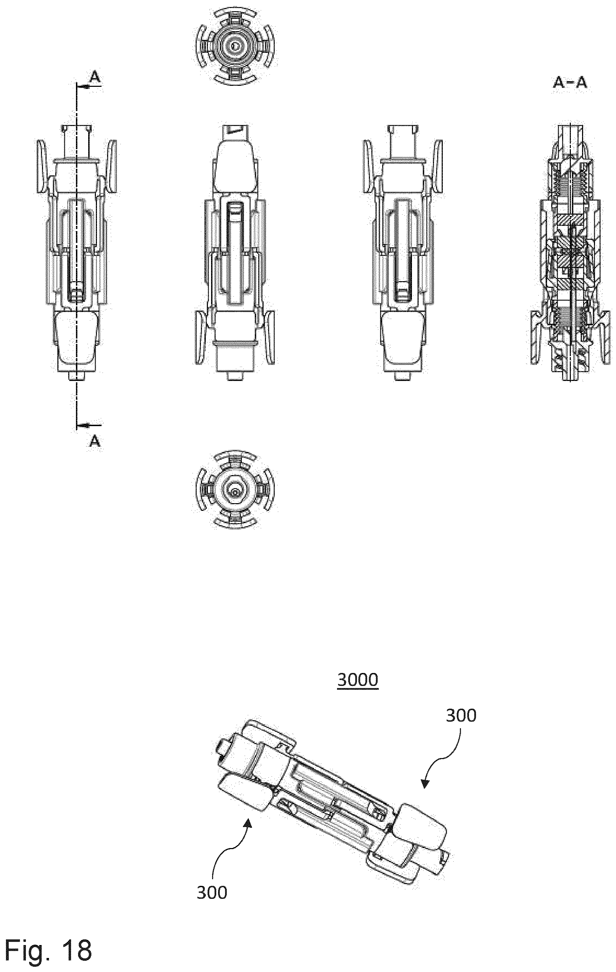

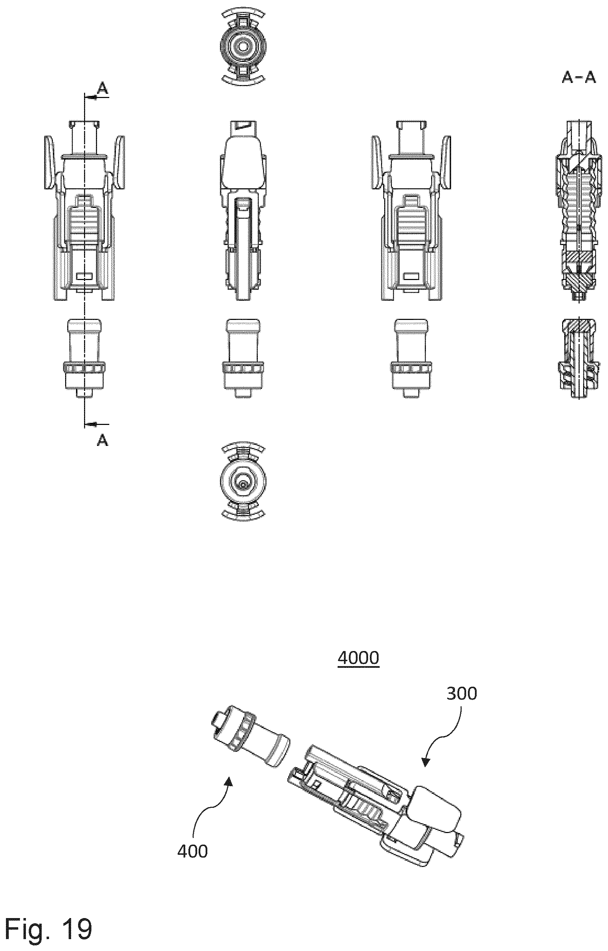

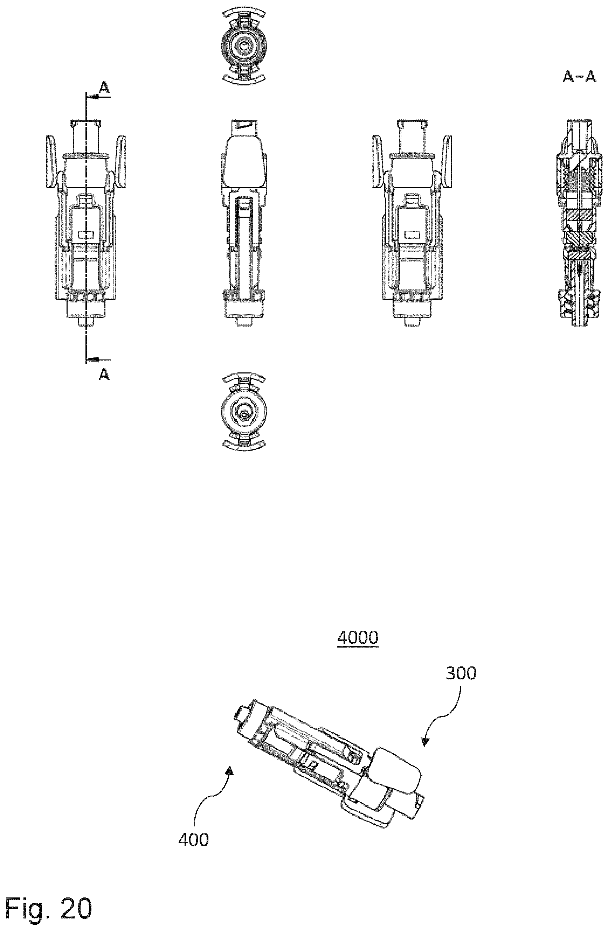

Fig. 13 zeigt ergänzend eine Übersicht der Außenansichten des Kupplungssystems gemäß den Figuren 1 bis 4, die Schnittansicht entlang der Schnittlinie A-A sowie eine perspektivische Ansicht im dekonnektierten Zustand. Analog hierzu zeigt Fig. 14 eine Übersicht gemäß Fig. 13 in einem konnektierten Zustand. Zu den Figuren 5 bis 7 zeigen die Figuren 15 und 16, zu den Figuren 8 bis 10 die Figuren 17 und 18 sowie zu den Figuren 11 und 12 die Figuren 19 und 20 die jeweiligen Übersichten jeweils für den dekonnektierten und konnektierten Zustand. Hieraus ergeben sich weitere Ausgestaltungsmerkmale der beschriebenen Ausführungsformen.

Die Erfindung ist nicht auf die beschriebenen Ausführungsformen beschränkt. Insbesondere sind bestimmte Merkmale einer Ausführungsform grundsätzlich auch auf andere Ausführungsformen anwendbar, sofern sich dies nicht vernünftigerweise ausschließt. Auch wenn die näher beschriebenen Kupplungssysteme jeweils baugleiche Kupplungselemente aufweisen, können auch komplementäre Kupplungselemente verwendet werden. Beispielsweise kann ein Kupplungssystem ein Kupplungselement gemäß der ersten Ausführungsform sowie ein Kupplungselement gemäß der zweiten Ausführungsform umfassen, wobei sich die Kupplungselemente hier lediglich im Aufbau Ihrer Dichtelemente unterscheiden, ohne dass dies ihrer Verbindung zum Fluidaustausch entgegensteht. Auch ist der vorstehend beschriebene Aufsatz nicht auf eine Verbindung mit dem Kupplungselement gemäß der dritten Ausführungsform beschränkt, sondern kann auch mit den anderen Kupplungselementen und Varianten hiervon verbindbar sein.

### Liste der Bezugszeichen:

- 10: Kupplungsgehäuse
- 10a: Freisparung
- 10b: vorragender Kupplungsgehäuseabschnitt
- 11: Dornaufnahme
- 12: Fluidanschluss
- 13: Kupplungsseite
- 20: Dichtelementaufnahme
- 21: Dichtelement
- 22: Dichtfläche
- 23: Dichtelement
- 25: komprimierbares Dichtelement
- 26: Befestigungsabschnitt
- 27: Befestigungselement
- 28: Befestigungselementabschnitt
- 24: fluiddichte Kammer
- 30: Dorn
- 31: vordere Fluidöffnung
- 32: hintere Fluidöffnung
- 33: Dornspitze
- 100: Kupplungselement
- 200: Kupplungselement
- 300: Kupplungselement
- 400: Aufsatz
- 410: Aufsatzgehäuse
- 411: Fluidkanal
- 412: Fluidanschluss
- 413: Kupplungsseite
- 421: Dichtelement
- 426: Befestigungsabschnitt
- 1000: Kupplungssystem
- 2000: Kupplungssystem
- 3000: Kupplungssystem
- 4000: Kupplungskit

## Patentansprüche

1. Kupplungselement (100, 200, 300) für ein Paar Kupplungselemente für ein gemeinsames geschlossenes Fluidtransfersystem, umfassend:
ein Kupplungsgehäuse (10) mit einer Längsachse und mit einem Fluidanschluss (12) und einer Kupplungsseite (13),
eine in dem Kupplungsgehäuse (10) angeordnete Dichtelementaufnahme (20) mit zumindest einer fluiddichten Kammer (24), die durch zumindest ein in der Dichtelementaufnahme (20) angeordnetes Dichtelement (21) ausgebildet ist, sowie einen in dem Kupplungsgehäuse (10) in einer Dornaufnahme (11) angeordneten Dorn (30), zur fluiden Verbindung mit dem Fluidanschluss (12), **dadurch gekennzeichnet, dass** der Dorn (30) in dem Kupplungsgehäuse (10) exzentrisch angeordnet ist.

2. Kupplungselement (100, 200, 300) nach Anspruch 1,
wobei die Dichtelementaufnahme (20) in einer Richtung entlang des Dorns (30) relativ zum Dorn (30) bewegbar ist und/oder der Dorn (30) in seiner Längsrichtung relativ zur Dichtelementaufnahme (20) bewegbar ist, und
wobei der Dorn (30) ausgehend von einer der Dornaufnahme (11) abgewandten Dornspitze (33) zumindest eine vordere Fluidöffnung (31) und eine hintere Fluidöffnung (32) umfasst,
wobei die vordere Fluidöffnung (31) so positioniert ist, dass sie in einer der Dornaufnahme (11) zugewandten Bewegungsendposition der Dichtelementaufnahme (20) und/oder in einer der Kupplungsseite (13) zugewandten Bewegungsendposition des Dorns (30) über die der Dornaufnahme (11) abgewandten Seite der fluiddichten Kammer (24) hinausragt und in einer der Dornaufnahme (11) abgewandten Bewegungsendposition der Dichtelementaufnahme (20) und/oder in einer der Kupplungsseite (13) abgewandten Bewegungsendposition des Dorns (30) in der fluiddichten Kammer (24) angeordnet ist.

3. Kupplungselement (100, 200, 300) nach Anspruch 1 oder 2, wobei die zumindest eine hintere Fluidöffnung (32) in einer der Dornaufnahme (11) zugewandten Bewegungsendposition der Dichtelementaufnahme (20) und/oder in einer Kupplungsseite (13) zugewandten Bewegungsendposition des Dorns (30) in der fluiddichten Kammer (24) angeordnet ist.

4. Kupplungselement (200, 300) nach einem der Ansprüche 1 bis 3, wobei die fluiddichte Kammer (24) aus zwei Dichtelementen (21, 23) gebildet ist, wobei das der Dornaufnahme (11) zugewandte Dichtelement (23) bevorzugt mit dem Dorn (30) in positionsfester Beziehung steht und die Dichtelementaufnahme (20) hierzu relativ zum Dorn (30) bewegbar ist.

5. Kupplungselement (300) nach einem der Ansprüche 1 bis 4, wobei das Kupplungsgehäuse (10) ein komprimierbares Dichtelement (25) umfasst, das die Dornaufnahme (11) mit der Dichtelementaufnahme (20) fluiddicht verbindet.

6. Kupplungselement (100, 200, 300) nach einem der Ansprüche 1 bis 5, wobei das Kupplungselement (100, 200, 300) in einer zur Erstreckungsrichtung des Dorns (30) radialen Umfangsfläche in Umfangsrichtung zumindest zwei Befestigungsabschnitte (26) aufweist, die jeweils für den Eingriff eines Befestigungselements (27) ausgebildet sind, und wobei die radiale Umfangsfläche in den in Umfangsrichtung zwischen den Befestigungsabschnitten (26) liegenden Bereichen Befestigungselementabschnitte (28) ausbildet, die jeweils zumindest ein Befestigungselement (27) aufweisen, wobei vorzugsweise die Befestigungsabschnitte (26) durch die Dichtelementaufnahme (20) gebildet werden, die mittels Freisparungen (10a) im Kupplungsgehäuse (10), die zur Kupplungsseite (13) hin offen sind, von der Kupplungsseite (13) aus zugänglich sind, wobei insbesondere die Befestigungselementabschnitte (28) zu den Befestigungsabschnitten (26) korrespondieren.

7. Kupplungselement (100, 200, 300) nach Anspruch 6, wobei das zumindest eine Befestigungselement (27) des jeweiligen Befestigungselementabschnitts (28) durch die Dichtelementaufnahme (20) gebildet wird, wobei vorzugsweise die Befestigungselemente (27) der Dichtelementaufnahme (20) über das Kupplungsgehäuse (10) in einem Befestigungsabschnitt (26) fixierbar sind.

8. Kupplungselement (100, 200, 300) nach Anspruch 6 oder 7, wobei die Befestigungsabschnitte (26) und die Befestigungselementabschnitte (28) komplementär zueinander ausgebildet sind, so dass bei einer Verbindung mit einem komplementären oder baugleichen Kupplungselement (100, 200, 300) eine fluiddichte Verbindung geschaffen wird.

9. Kupplungselement (100, 200, 300) nach einem der Ansprüche 6 bis 8, wobei das Kupplungsgehäuse (10) gegenüber der Dichtelementaufnahme (20) erst dann relativ bewegbar ist, wenn das jeweilige Befestigungselement (27) bei Kopplung mit einem komplementären oder baugleichen Kupplungselement (100, 200, 300) mit dem Befestigungsabschnitt (26) des anderen Kupplungselements (100, 200, 300) einen Kupplungszustand ausbildet.

10. Kupplungssystem (1000, 2000, 3000) mit zwei Kupplungselementen (100, 200, 300) nach einem der Ansprüche 1 bis 9.

11. Kupplungssystem (1000, 2000, 3000) nach Anspruch 10, wobei die Kupplungselemente (100, 200, 300) jeweils miteinander komplementär, insbesondere baugleich, sind und durch einen relativen Winkelversatz um ihre Längsachse, bevorzugt durch einen relativen Winkelversatz um 90°, ohne Adapter koppelbar sind.

12. Kupplungssystem (1000, 2000, 3000) nach Anspruch 10 oder 11, wobei zumindest eine der jeweiligen vorderen Fluidöffnungen (31), insbesondere beide vorderen Fluidöffnungen (31), in einem Kupplungszustand, in dem sich die jeweiligen Dichtelementaufnahmen (20) in einer zu der jeweiligen Dornaufnahme (11) zugewandten Bewegungsendposition befinden, in der fluiddichten Kammer (24) des jeweils anderen Kupplungselements (100, 200, 300) angeordnet ist und mit der jeweiligen hinteren Fluidöffnung (32) des anderen Kupplungselements (100, 200, 300), das vorzugsweise im Kupplungszustand ebenfalls in dieser fluiddichten Kammer (24) angeordnet ist, in fluider Verbindung steht.

13. Kupplungssystem (1000, 2000, 3000) nach einem der Ansprüche 10 bis 12, wobei die der Kupplungsseite (13) zugewandten Dichtflächen (22) der Dichtelemente (21) der Kupplungselemente (100, 200, 300) im Kupplungszustand in Kontakt sind und der Kontakt bei einer Entkopplung erst aufgehoben wird, wenn die jeweiligen Dichtelementaufnahmen (20) in einer der jeweiligen Dornaufnahme (11) abgewandten Bewegungsendposition sind.

14. Kupplungskit (4000) mit einem Aufsatz (400) für ein Kupplungselement nach einem der Ansprüche 1 bis 9, wobei der Aufsatz (400) einen Befestigungsabschnitt (426) umfasst, der so ausgebildet ist, dass er in einer der Dornaufnahme (11) zugewandten Bewegungsendposition der Dichtelementaufnahme (20) im Kupplungselement fixierbar ist, und weitere Bestandteile ausgewählt aus einem Kupplungselement (100, 200, 300) nach einem der Ansprüche 1 bis 9 oder einem Kupplungssystem (1000, 2000, 3000) nach einem der Ansprüche 10 bis 13.

## Claims

1. Coupling element (100, 200, 300) for a pair of coupling elements for a shared closed fluid transfer system, comprising:
a coupling housing (10) having a longitudinal axis and having a fluid port (12) and a coupling face (13),
a sealing element receptacle (20) arranged in the coupling housing (10) and having at least one fluid-tight chamber (24), formed by at least one sealing element (21) arranged in the sealing element receptacle (20), and a mandrel (30) arranged in the coupling housing (10) in a mandrel receptacle (11), for fluid connection to the fluid port (12), **characterised in that** the mandrel is arranged eccentrically in the coupling housing (10).

2. Coupling element (100, 200, 300) according to claim 1,
wherein the sealing element receptacle (20) is movable in a direction along the mandrel (30) relative to the mandrel (30) and/or the mandrel (30) is movable in its longitudinal direction relative to the sealing element receptacle (20), and
wherein the mandrel (30) comprises at least a front fluid opening (31) and a rear fluid opening (32), starting from a mandrel tip (33) facing away from the mandrel receptacle (11),
wherein the front fluid opening (31) is positioned in such a way that, in a movement end position of the sealing element receptacle (20) facing the mandrel receptacle (11) and/or in a movement end position of the mandrel (30) facing the coupling face (13), it projects beyond the face of the fluid-tight chamber (24) facing away from the mandrel receptacle (11), and, in a movement end position of the sealing element receptacle (20) facing away from the mandrel receptacle (11) and/or in a movement end position of the mandrel (30) facing away from the coupling face (13), it is arranged in the fluid-tight chamber (24).

3. Coupling element (100, 200, 300) according to either claim 1 or claim 2, NB:MBO
wherein, in a movement end position of the sealing element receptacle (20) facing the mandrel receptacle (11) and/or in a movement end position of the mandrel (30) facing a coupling face (13), the at least one rear fluid opening (32) is arranged in the fluid-tight chamber (24).

4. Coupling element (200, 300) according to any of claims 1 to 3, wherein the fluid-tight chamber (24) is formed from two sealing elements (21, 23), the sealing element (23) facing the mandrel receptacle (11) preferably being in a fixed positional relationship with the mandrel (30), and the sealing element receptacle (20) being movable relative to the mandrel (30) for this purpose.

5. Coupling element (300) according to any of claims 1 to 4, wherein the coupling housing (10) comprises a compressible sealing element (25) which connects the mandrel receptacle (11) to the sealing element receptacle (20) in a fluid-tight manner.

6. Coupling element (100, 200, 300) according to any of claims 1 to 5, wherein the coupling element (100, 200, 300) has, in the circumferential direction in a circumferential surface radial with respect to the direction of extent of the mandrel (30), at least two fastening portions (26), each configured for the engagement of a fastening element (27), and wherein the radial circumferential surface forms, in the regions lying between the fastening portions (26) in the circumferential direction, fastening element portions (28) which each comprise at least one fastening element (27), the fastening portions (26), which are accessible from the coupling face (13) via clearances (10a) in the coupling housing (10) which are open towards the coupling face (13), preferably being formed by the sealing element receptacle (20), in particular the fastening element portions (28) corresponding to the fastening portions (26).

7. Coupling element (100, 200, 300) according to claim 6, wherein the at least one fastening element (27) of each fastening element portion (28) is formed by the sealing element receptacle (20), the fastening elements (27) of the sealing element receptacle (20) preferably being fixable in a fastening portion (26) via the coupling housing (10).

8. Coupling element (100, 200, 300) according to either claim 6 or claim 7, wherein the fastening portions (26) and the fastening element portions (28) are formed mutually complementary in such a way that, upon connection to a complementary or identically constructed coupling element (100, 200, 300), a fluid-tight connection is created.

9. Coupling element (100, 200, 300) according to any of claims 6 to 8, wherein the coupling housing (10) is movable relative to the sealing element receptacle (20) only when the associated fastening element (27), upon coupling to a complementary or identically constructed coupling element (100, 200, 300), establishes a coupled state with the fastening portion (26) of the other coupling element (100, 200, 300).

10. Coupling system (1000, 2000, 3000) comprising two coupling elements (100, 200, 300) according to any of claims 1 to 9.

11. Coupling system (1000, 2000, 3000) according to claim 10, wherein the coupling elements (100, 200, 300) are in each case mutually complementary to each other, in particular constructed identically, and can be coupled without an adapter by means of a relative angular offset about their longitudinal axis, preferably a relative angular offset of 90°.

12. Coupling system (1000, 2000, 3000) according to either claim 10 or claim 11, wherein, in a coupled state in which the respective sealing element receptacles (20) are located in a movement end position facing the associated mandrel receptacle (11), at least one of the associated front fluid openings (31), in particular both front fluid openings (31), is arranged in the fluid-tight chamber (24) of the other coupling element (100, 200, 300) and is fluidically connected to the associated rear fluid opening (32) of the other coupling element (100, 200, 300), which in the coupled state is preferably likewise arranged in this fluid-tight chamber (24).

13. Coupling system (1000, 2000, 3000) according to any of claims 10 to 12, wherein the sealing faces (22), facing the coupling face (13), of the sealing elements (21) of the coupling elements (100, 200, 300) are in contact in the coupled state, and during decoupling the contact is broken only when the respective sealing element receptacles (20) are in a movement end position facing away from the associated mandrel receptacle (11).

14. Coupling kit (4000), comprising an attachment (400) for a coupling element according to any of claims 1 to 9, the attachment (400) comprising a fastening portion (426) formed so as to be fixable in the coupling element in a movement end position of the sealing element receptacle (20) facing the mandrel receptacle (11), and further components selected from a coupling element (100, 200, 300) according to any of claims 1 to 9 or a coupling system (1000, 2000, 3000) according to any of claims 10 to 13.

## Revendications

1. Élément d'accouplement (100, 200, 300) pour une paire d'éléments d'accouplement pour un système de transfert de fluide fermé commun, comprenant :
un boîtier d'accouplement (10) ayant un axe longitudinal et un raccord de fluide (12) et un côté d'accouplement (13) ;
un logement d'élément d'étanchéité (20) disposé dans le boîtier d'accouplement (10) comportant au moins une chambre étanche aux fluides (24) qui est formée par au moins un élément d'étanchéité (21) disposé dans le logement d'élément d'étanchéité (20), ainsi qu'une broche (30) disposée dans le boîtier d'accouplement (10) dans un logement de broche (11) pour être fluidiquement reliée au raccord de fluide (12), **caractérisé en ce que** la broche est disposée dans le boîtier d'accouplement (10) de manière excentrée.

2. Élément d'accouplement (100, 200, 300) selon la revendication 1,
dans lequel le logement d'élément d'étanchéité (20) peut être déplacé dans une direction le long de la broche (30) par rapport à la broche (30) et/ou la broche (30) peut être déplacée dans sa direction longitudinale par rapport au logement d'élément d'étanchéité (20), et
dans lequel la broche (30) comprend, en partant d'une pointe de broche (33) opposée au logement de broche (11), au moins une ouverture de fluide avant (31) et une ouverture de fluide arrière (32),
dans lequel l'ouverture de fluide avant (31) est positionnée de telle sorte que, dans une position de fin de mouvement du logement d'élément d'étanchéité (20) faisant face au logement d'élément d'étanchéité (20) et/ou dans une position de fin de mouvement de la broche (30) faisant face au logement de broche (11), elle fait saillie au-delà du côté de la chambre étanche aux fluides (24) opposé au logement de broche (11), et dans une position de fin de mouvement opposée au logement de broche (20) et/ou dans une position de fin de mouvement de la broche (30) opposée au côté d'accouplement (13), elle est disposée dans la chambre étanche aux fluides (24).

3. Élément d'accouplement (100, 200, 300) selon la revendication 1 ou 2, dans lequel, dans une position de fin de mouvement du logement d'élément d'étanchéité (20) faisant face au logement de broche (11) et/ou dans une position de fin de mouvement de la broche (30) faisant face à un côté d'accouplement (13), ladite au moins une ouverture de fluide arrière (32) est disposée dans la chambre étanche aux fluides (24).

4. Élément d'accouplement (200, 300) selon l'une des revendications 1 à 3, dans lequel la chambre étanche aux fluides (24) est formée de deux éléments d'étanchéité (21, 23), dans lequel l'élément d'étanchéité (23) faisant face au logement de broche (11) est de préférence en relation de position fixe avec la broche (30) et le logement d'élément d'étanchéité (20) peut être déplacé jusqu'à celui-ci par rapport à la broche (30).

5. Élément d'accouplement (300) selon l'une des revendications 1 à 4, dans lequel le boîtier d'accouplement (10) comprend un élément d'étanchéité compressible (25) qui relie le logement de broche (11) au logement d'élément d'étanchéité (20) de manière étanche aux fluides.

6. Élément d'accouplement (100, 200, 300) selon l'une des revendications 1 à 5, dans lequel l'élément d'accouplement (100, 200, 300) comporte, dans une surface circonférentielle radiale par rapport à la direction d'extension de la broche (30) dans une direction circonférentielle, au moins deux parties de fixation (26) qui sont respectivement formées pour la prise d'un élément de fixation (27), et dans lequel la surface circonférentielle radiale dans les zones situées dans la direction circonférentielle entre les parties de fixation (26) forme des parties d'élément de fixation (28) qui comportent respectivement au moins un élément de fixation (27), dans lequel les parties de fixation (26) sont de préférence formées par le logement d'élément d'étanchéité (20) qui, au moyen d'évidements (10a) dans le boîtier d'accouplement (10) ouverts vers le côté d'accouplement (13), sont accessibles à partir du côté d'accouplement (13), dans lequel les parties d'élément de fixation (28) correspondent en particulier aux parties de fixation (26).

7. Élément d'accouplement (100, 200, 300) selon la revendication 6, dans lequel ledit au moins un élément de fixation (27) de la partie d'élément de fixation (28) respective est formé par le logement d'élément d'étanchéité (20), dans lequel les éléments de fixation (27) du logement d'élément d'étanchéité (20) peuvent de préférence être fixés dans une partie de fixation (26) par l'intermédiaire du boîtier d'accouplement (10).

8. Élément d'accouplement (100, 200, 300) selon la revendication 6 ou 7, dans lequel les parties de fixation (26) et les parties d'élément de fixation (28) sont formées de manière complémentaire les unes aux autres, de telle sorte qu'une liaison étanche aux fluides est réalisée lors d'un raccordement à un élément d'accouplement complémentaire ou structurellement identique (100, 200, 300).

9. Élément d'accouplement (100, 200, 300) selon l'une des revendications 6 à 8, dans lequel le boîtier d'accouplement (10) n'est mobile de façon relative par rapport au logement d'élément d'étanchéité (20) que si l'élément de fixation (27) respectif forme un état d'accouplement avec la partie de fixation (26) de l'autre élément d'accouplement (100, 200, 300) lors d'un accouplement à un élément d'accouplement complémentaire ou structurellement identique (100, 200, 300).

10. Système d'accouplement (1000, 2000, 3000) comportant deux éléments d'accouplement (100, 200, 300) selon l'une des revendications 1 à 9.

11. Système d'accouplement (1000, 2000, 3000) selon la revendication 10, dans lequel les éléments d'accouplement (100, 200, 300) sont respectivement complémentaires les uns des autres, en particulier structurellement identiques, et peuvent être accouplés sans adaptateur par un décalage angulaire relatif autour de leur axe longitudinal, de préférence par un décalage angulaire relatif de 90°.

12. Système d'accouplement (1000, 2000, 3000) selon la revendication 10 ou 11, dans lequel au moins l'une des ouvertures de fluide avant (31) respectives, en particulier les deux ouvertures de fluide avant (31), est disposée dans un état d'accouplement dans lequel les logements d'élément d'étanchéité (20) respectifs se trouvent dans une position de fin de mouvement faisant face au logement de broche (11) respectif, dans la chambre étanche aux fluides (24) de l'autre élément d'accouplement (100, 200, 300) respectif et est en liaison fluidique avec l'ouverture de fluide arrière (32) respective de l'autre élément d'accouplement (100, 200, 300) qui est de préférence également disposé dans cette chambre étanche aux fluides (24) à l'état d'accouplement.

13. Système d'accouplement (1000, 2000, 3000) selon l'une des revendications 10 à 12, dans lequel les surfaces d'étanchéité (22) des éléments d'étanchéité (21) des éléments d'accouplement (100, 200, 300) faisant face au côté d'accouplement (13) sont en contact à l'état d'accouplement et le contact n'est supprimé lors d'un désaccouplement que si les logements d'élément d'étanchéité (20) respectifs sont dans une position de fin de mouvement opposée au logement de broche (11) respectif.

14. Kit d'accouplement (4000) comportant un accessoire (400) pour un élément d'accouplement selon l'une des revendications 1 à 9, dans lequel l'accessoire (400) comprend une partie de fixation (426) qui est configurée de telle sorte qu'elle peut être fixée dans l'élément d'accouplement dans une position de fin de mouvement du logement d'élément d'étanchéité (20) faisant face au logement de broche (11), et des composants supplémentaires choisis parmi un élément d'accouplement (100, 200, 300) selon l'une des revendications 1 à 9 ou un système d'accouplement (1000, 2000, 3000) selon l'une des revendications 10 à 13.
